## Europäisches Patentamt
## European Patent Office
## Office européen des brevets

(19)

(11) Numéro de publication: **0 252 774**
**A1**

(12)

# DEMANDE DE BREVET EUROPEEN

(21) Numéro de dépôt: **87401050.7**

(22) Date de dépôt: **07.05.87**

(51) Int. Cl.³: **C 12 N 15/00**
**C 12 N 1/16, C 12 N 1/18**
**C 12 N 9/28**

(30) Priorité: **09.05.86 FR 8606703**

(43) Date de publication de la demande:
**13.01.88 Bulletin 88/2**

(84) Etats contractants désignés:
**AT BE CH DE ES FR GB GR IT LI LU NL SE**

(71) Demandeur: **TRANSGENE S.A.**
**16, rue Henri Régnault**
**F-92400 Courbevoie(FR)**

(72) Inventeur: **Gloeckler, Rémi**
**22, rue de Stockholm**
**F-67000 Strasbourg(FR)**

(72) Inventeur: **Loison, Gérard**
**19, rue du Faubourg National**
**F-67000 Strasbourg(FR)**

(72) Inventeur: **Lemoine, Yves**
**4, rue des Alisiers**
**F-67000 Strasbourg(FR)**

(74) Mandataire: **Warcoin, Jacques et al,**
**Cabinet Régimbeau 26, avenue Kléber**
**F-75116 Paris(FR)**

(54) Souches de saccharomyces produisant de l'alpha-amylase.

(57) L'invention concerne une souche de *Saccharomyces* caractérisée en ce qu'elle comporte un bloc d'expression de l'exo-alpha-amylase intégré dans un chromosome ou bien inséré sur un vecteur extrachromosomique.

EP 0 252 774 A1

# SOUCHES DE SACCHAROMYCES PRODUISANT DE L'ALPHA-AMYLASE

La présente invention décrit des procédés permettant de faire sécréter une exo-alpha-amylase thermorésistante par différentes souches de levure du genre Saccharomyces.

Les levures Saccharomyces sécrètent naturellement des protéines dans le milieu de culture, ces protéines sont synthétisées sous forme de précurseur dont la maturation en exoprotéines se fait le long du chemin de sécrétion (1). Il est possible de faire sécréter par la levure des enzymes étrangères d'intérêt industriel, comme par exemple des alpha-amylases d'origines diverses/(levure Saccharomycopsis (2), blé (3), souris (4)/ ou des alpha-amyloglucosidases /(Aspergillus (5), Saccharomycopsis (6)/, etc.

Les souches de Bacillus licheniformis produisent des exo-alpha-amylases thermostables qui sont utilisées dans les procédés de liquéfaction de l'amidon ou dans ceux conduisant à l'obtention de glucose à partir d'amidon.

L'objet de la présente invention est de faire produire une telle exo-alpha-amylase thermostable par des levures du genre Saccharomyces.

Plus particulièrement, la présente invention concerne de nouvelles souches de Saccharomyces, caractérisées en ce qu'elles comportent un bloc d'expression de l'exo-alpha-amylase intégré dans un chromosome ou bien inséré sur un vecteur extrachromosomique.

Parmi les exo-alpha-amylases selon l'invention, il faut citer plus particulièrement les amylases thermostables de Bacillus licheniformis.

Le bloc d'expression de l'exo-alpha-amylase a, de préférence, la structure suivante :

```
          Pr      Pre  Pro   alpha-amylase
   ───┼────────────┼────┼──────────────────────┼───   .
```

Pr est un promoteur de levure,

alpha-amylase est la séquence codant pour la partie mature de l'alpha-amylase,

Pre Pro est la séquence pré pro du gène de la phéromone alpha-1.

Le promoteur de levure peut être, par exemple, le promoteur du gène PGK, quant à l'extrémité 3' de la séquence, elle peut comporter un terminateur de levure tel que le terminateur du gène PGK.

Lorsque ce bloc d'expression est intégré dans un chromosome, la technique d'intégration étant une recombinaison, on intègrera de préférence le bloc d'expression dans un gène conférant un phénotype qui peut aisément être mis en évidence, par exemple le gène HIS, l'intégration du bloc d'expression conduisant au phénotype his⁻ qui peut être mis en évidence.

Lorsque le bloc d'expression est inséré dans un vecteur extrachromosomique, il s'agira de préférence d'un plasmide comportant une origine de réplication de levure, par exemple l'origine de réplication du plasmide 2µ.

Ce type de vecteur comportera, en outre, de préférence un gène marqueur de sélection tel que $ura_3^+$ ou Leu$_2^+$ qui assurent la complémentation pour l'uracile ou la leucine. Ces vecteurs peuvent également être des plasmides navettes et comporter une origine de réplication dans les bactéries, par exemple celle du plasmide pBR322, et un ou plusieurs gènes de sélection dans les bactéries, tels que Amp$^r$ par exemple.

Dans certains cas, il peut être nécessaire d'utiliser, lors de la cotransformation, un vecteur conférant un phénotype dominant, comme dans le cas des souches industrielles prototrophes. Parmi les phénotypes dominants commodes, il faut citer la résistance au G418.

Parmi les souches industrielles transformées, il faut citer, en particulier, les souches polyploïdes de boulangerie telles que TGF1 ou les souches de brasserie telles que S. uvarum.

Bien entendu, les souches selon l'invention permettent, tout d'abord, la préparation d'une exo-alpha-amylase thermostable.

C'est pourquoi la présente invention concerne un procédé de préparation d'une alpha-amylase thermostable, caractérisé en ce qu'on cultive sur un milieu de culture une souche de Saccharomyces selon l'invention et on récupère l'enzyme dans le milieu de culture.

Mais, l'intérêt de cette expression hétérologue est multiple.

Tout d'abord, les levures selon l'invention produisant l'exo-alpha-amylase sont facilement identifiables sur milieu solide contenant de l'amidon.

En effet, l'amidon au contact de l'iode ($I_2$) prend une coloration bleue, or les levures selon l'invention, qui produisent l'exo-alpha-amylase dans le milieu de culture, dégradent l'amidon, il s'ensuit un halo de décoloration autour des colonies exprimant cette activité amylolytique. On peut ainsi très facilement distinguer les levures transformées des levures Saccharomyces de type sauvage qui ne sécrètent pas d'activité alpha-amylasique.

D'autre part, les levures produisant l'exo-alpha-amylase peuvent à leur tour servir comme hôte de transformation.

'L'introduction du bloc d'expression génétique de l'exo-alpha-amylase en un exemplaire dans un site préalablement caractérisé du génome de la cellule hôte, suivant les techniques connues de l'homme de l'art sous le vocable anglais "gene disruption" (7) et précisées ci-après, permet de "marquer" la souche réceptrice. Si on veut par ces mêmes techniques introduire une nouvelle séquence d'ADN à la place du bloc d'expression de l'exo-alpha-amylase, il est possible d'utiliser cette souche transformée exprimant l'exo-alpha-amylase comme réceptrice pour la transformation. Les colonies issues des cellules ayant échangé les séquences d'expression d'ADN sont facilement repérables car elles ont perdu la capacité de former le halo de décoloration de l'amidon en présence d'iode. Même si la fréquence d'échange est faible et nécessite l'analyse de 100 à 1 000 clones transformés dans des expériences de cotransformation, la détection par l'absence de halo permet de repérer d'emblée les candidats intéressants et donc de limiter l'analyse aux clones n'exprimant plus l'alpha-amylase.

En particulier, il n'est pas nécessaire que la nouvelle séquence code pour une nouvelle activité enzymatique ni de procéder à des recherches systématiques par hybridation d'acide nucléique pour discriminer les candidats recherchés du reste des clones transformés.

C'est pourquoi l'invention concerne l'application des levures selon l'invention pour l'intégration d'une séquence d'ADN déterminée dans ladite souche, caractérisée en ce que le vecteur d'intégration comporte la séquence d'ADN à intégrer entre des séquences assurant l'échange avec le bloc d'expression de l'exo-alpha-amylase ou une partie essentielle de ce bloc, et en ce que l'on sélectionne les souches ne produisant plus de halo de décoloration en présence d'iode ($I_2$) et d'amidon.

En troisième lieu, l'activité de l'alpha-amylase de B. licheniformis se caractérise par le clivage des liaisons alpha$_{1-4}$ de l'amylose et de l'amylopectine libérant essentiellement des dextrines. L'action conjointe de l'alpha-amylase et de l'alpha-amyloglucosidase résulte dans une synergie totale conduisant à une dégradation efficace de l'amidon essentiellement en résidus glucoses directement assimilables par la levure.

En dernier lieu, l'utilisation d'une levure de boulangerie _Saccharomyces cerevisiae_ dont le patrimoine génétique contient le nouveau caractère génétique conduisant à l'expression et à la sécrétion d'une telle alpha-amylase permettrait d'obtenir un produit de panification dont le rassissement serait freiné par l'action bénéfique d'une activité alpha-amylase thermostable résiduelle (8).

Les exemples ci-après sont destinés à illustrer d'autres caractéristiques et avantages de la présente invention.

## EXEMPLE 1

## CLONAGE DU GENE DE L'ALPHA-AMYLASE DE BACILLUS LICHENIFORMIS DANS LE BACTERIOPHAGE λ

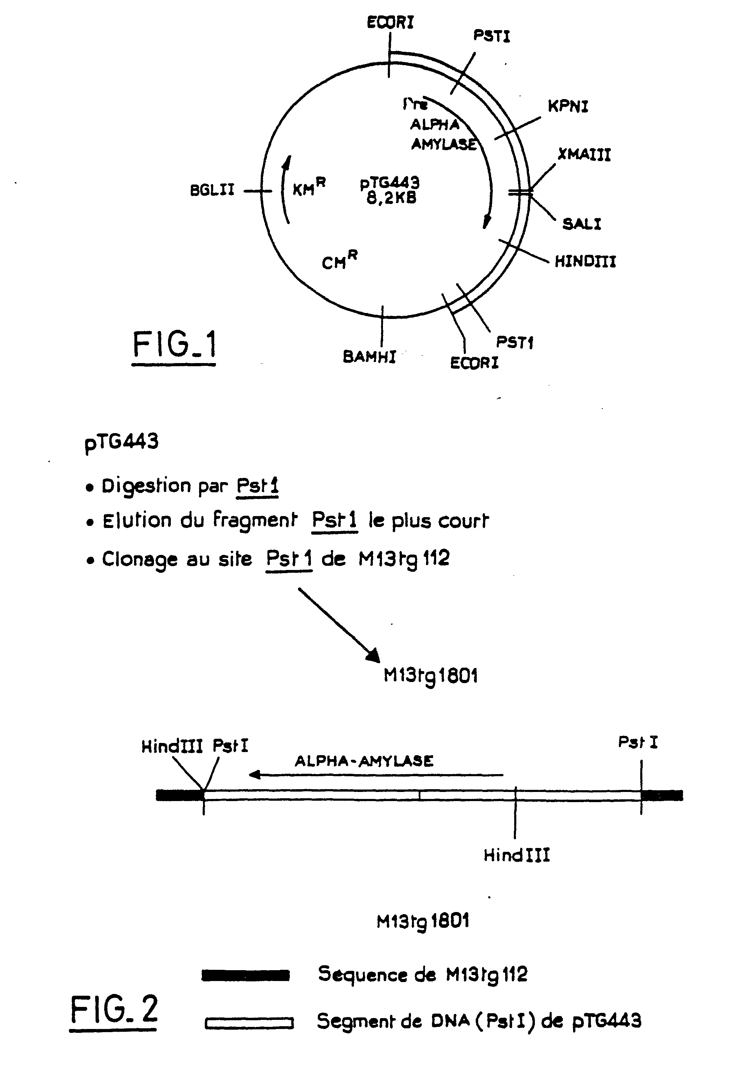

On a complètement digéré, par EcoRI, 1 µg d'ADN génomique de B. licheniformis NCIB6346 et 1 µg d'ADN du bactériophage λ607, puis lié covalemment ces ADNs à l'aide de la T4 ADN ligase. 50 µg du produit de ligation ont été empaquetés in vitro (9) dans les particules phagiques et les phages ainsi formés ont servi à transduire la souche indicatrice E. coli POP101. Sur les boîtes de transduction, on a coulé une couche de L-agar supplémenté avec 0,4 % (poids-volume) d'amidon soluble. Après incubation des boîtes une nuit à 37°C, on a exposé les boîtes aux vapeurs d'iode. l'amidon au contact de l'iode se colore. L'amidon dégradé par l'alpha-amylase fixe beaucoup moins l'iode. Un halo de décoloration présent autour d'une plage de lyse a permis de repérer des bactéries infectées qui produisent de l'alpha-amylase active. L'analyse de l'ADN du phage responsable de cette plage de lyse a révélé qu'il contenait un fragment EcoRI de 3,5 kb provenant de B. licheniformis et correspondant au gène de l'alpha-amylase en entier. Ce fragment a été recloné dans un vecteur navette E. coli-B. subtilis pBD64, décrit dans la littérature (10). Après transformation de la souche B. subtilis 1A289, on a pu isoler un plasmide qui dérive de l'addition du fragment EcoRI au site EcoRI de pBD64, ce plasmide a été appelé pTG443 (figure 1).

## EXEMPLE 2

## CONSTRUCTION D'UN VECTEUR D'EXPRESSION D'EXO-ALPHA-AMYLASE POUR LA LEVURE S. CEREVISIAE

Le fragment cloné dans pTG443 possède un site PstI situé au début du gène codant pour l'alpha-amylase et un autre site PstI situé en aval du signal d'arrêt de traduction de ce gène. Ce fragment PstI, portant l'essentiel de l'information pour l'alpha-amylase, a été cloné dans le site <u>Pst</u>I unique du phage M13TG112. Ce nouveau phage est appelé M13TG1801.

L'intérêt du clonage est de permettre de ressortir la séquence de l'alpha-amylase sous forme d'un fragment <u>Hind</u>III (figure 2). Ce fragment <u>Hind</u>III a été cloné dans le site <u>Hind</u>III du phage M13TG889 dont la construction est détaillée ci-après.

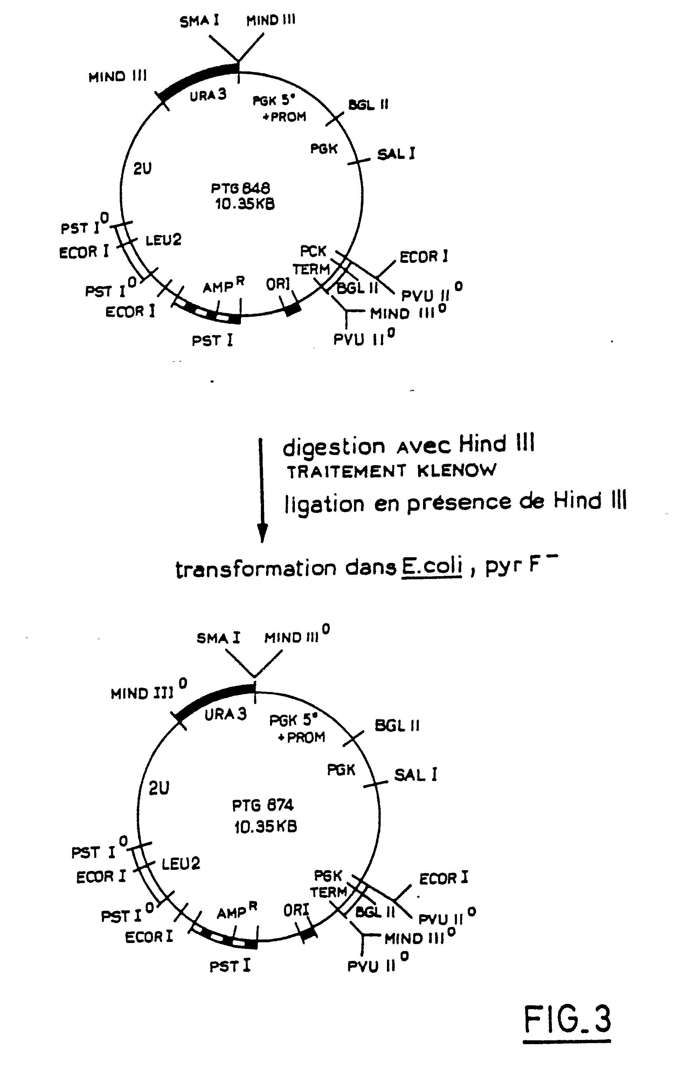

Le plasmide de départ est le pTG848 (identique au pTG849 décrit dans le brevet français 83 15716 à l'exception du gène ura3 dont l'orientation est inversée), il est constitué des fragments d'ADN suivants (figure 3) :

1) Le fragment <u>Eco</u>RI-<u>Hind</u>III d'environ 3,3 kb en provenance du plasmide pJDB207 (11). Le site <u>Hind</u>III correspond à la coordonnée 105 du plasmide 2μ forme B, le site <u>Eco</u>RI à la coordonnée 2243. Dans ce fragment le 2μ est de la forme B (12). Ce fragment permet la réplication autonome du plasmide dans la levure et la sélection du plasmide dans les souches ura3⁻ sur milieu dépourvu de source de pyrimidine.

2) Le fragment <u>Hind</u>III du gène URA3 (13).

3) Le grand fragment <u>Eco</u>RI (coordonnée 0)-<u>Sal</u>I (coordonnée 650) de pBR322 (14). Dans le site <u>Pvu</u>II de ce fragment a été inséré le fragment <u>Eco</u>RI-<u>Hind</u>III dont les extrémités ont été préalablement rendues franches par action de la Klenow en présence des 4 désoxyribonucléotides, fragment de 510 paires de bases et correspondant à la fin du gène PGK (15). L'extrémité <u>Eco</u>RI arasée du gène PGK, lorsque jointe à l'extrémité <u>Pvu</u>II de pBR322, régénère un site <u>Eco</u>RI.

4) Le fragment <u>Hind</u>III-<u>Sal</u>I (2,15 kb) du gène PGK (15).

Le plasmide pTG848 a été coupé par HindIII, les extrémités des deux fragments ainsi libérés sont rendues franches par traitement à la klenow en présence des 4 désoxyribonucléotides (16). Les deux fragments sont mis en ligation et, avant transformation, ce mélange de ligation est soumis à l'action de HindIII, ce qui permet d'éliminer toute forme de plasmide ayant conservé un (ou deux) site HindIII. On transforme la souche E. coli (pyrF) (17) et on sélectionne les transformés pour la résistance à l'ampicilline et pour le caractère pyrF$^+$. On obtient ainsi le plasmide pTG874 (figure 3) où les deux sites HindIII sont supprimés, l'orientation du gène URA3 donnant lieu à une transcription dans la même orientation que celle du gène de la phospho-glycérate kinase (PGK).

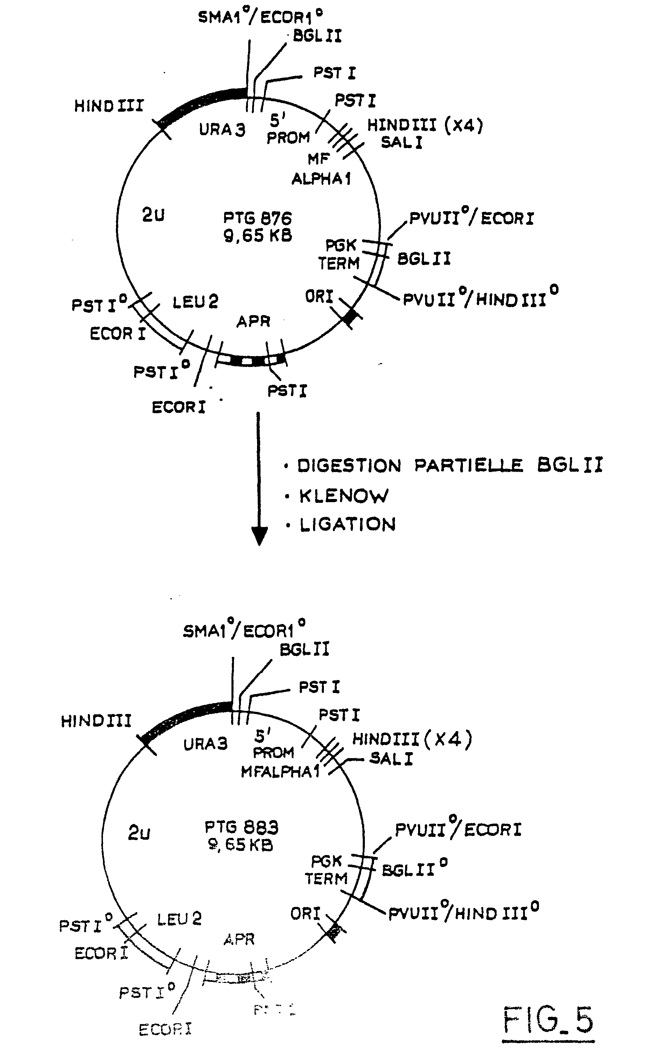

Le plasmide pTG874 est coupé par SmaI et SalI, le fragment de 8,6 kb est ensuite isolé à partir d'un gel d'agarose. Le plasmide pTG864, qui comprend le fragment EcoRI-SalI (environ 1,4 kb) du gène MFα1 cloné dans les mêmes sites de pBR322, est coupé par EcoRI. Les extrémités du plasmide ainsi linéarisé sont rendues franches par action de la klenow en présence des 4 nucléotides. Une digestion par l'enzyme SalI est ensuite effectuée et le fragment EcoRI (extrémité franche)-SalI correspondant au gène MFα1 est isolé. Ce dernier fragment est mis en ligation avec le morceau SmaI-SalI (8,6 kb) du pTG874 pour donner ainsi le plasmide pTG876 (figure 4).

Afin de supprimer le site BglII situé en aval du gène MFα1, on a digéré partiellement ce plasmide pTG876 par BglII, puis on a fait agir le fragment Klenow de la DNA polymérase de E. coli en présence des 4 désoxyribonucléotides et on a lié covalemment les molécules d'ADN sur elles-mêmes grâce à l'action de la DNA ligase du phage T4. Le plasmide pTG883 ne possède plus qu'un seul site BglII, situé en amont du promoteur du gène de la phéromone (figure 5).

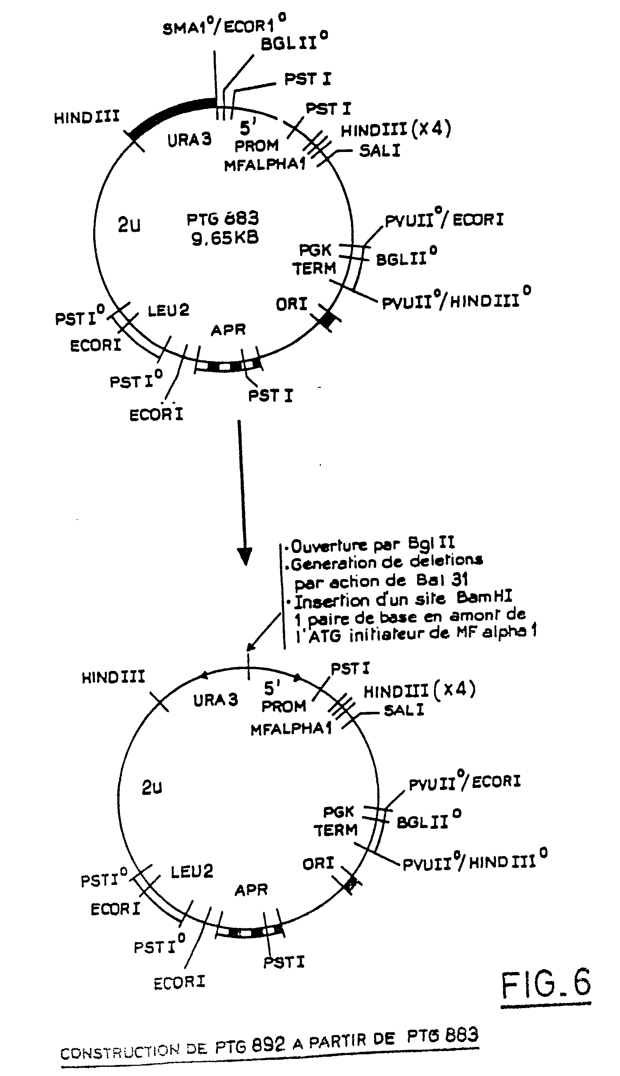

Le plasmide pTG883 (20 µg) a été linéarisé par digestion par BglII puis soumis à une digestion ménagée par la nucléase Bal31 (1,4 unités, 10 minutes - Boehringer Mannheim) suivant les techniques habituelles. L'ADN ainsi traité a été purifié par extractions successives au phénol et chloroforme-alcool isoamylique, puis une fraction (5 µg) a été soumise à l'action de la polymérase Klenow afin d'obtenir un maximum d'extrémités franches. L'ADN ainsi traité a été mis à "liguer" avec des "linkers" BamHI non phosphorylés (5'-CGGATCCG) en présence de la ligase du phage T4 suivant la technique décrite par Lathe et al. (18). Après transformation de E. coli 1106 pour la résistance à l'ampicilline, on a isolé un plasmide pTG892 (figure 6) délété pour la région 5' flanquant le gène MFα1, le linker BamHI étant localisé une base en amont de l'ATG suivant le schéma :

$$5' - \underline{CGGGATCCCG} \qquad A \qquad \underline{ATG\ AGA\ TTT\ ...\ ...}$$
$$\text{linker BamHI} \qquad \text{partie codante } MT\alpha_{1-1}$$

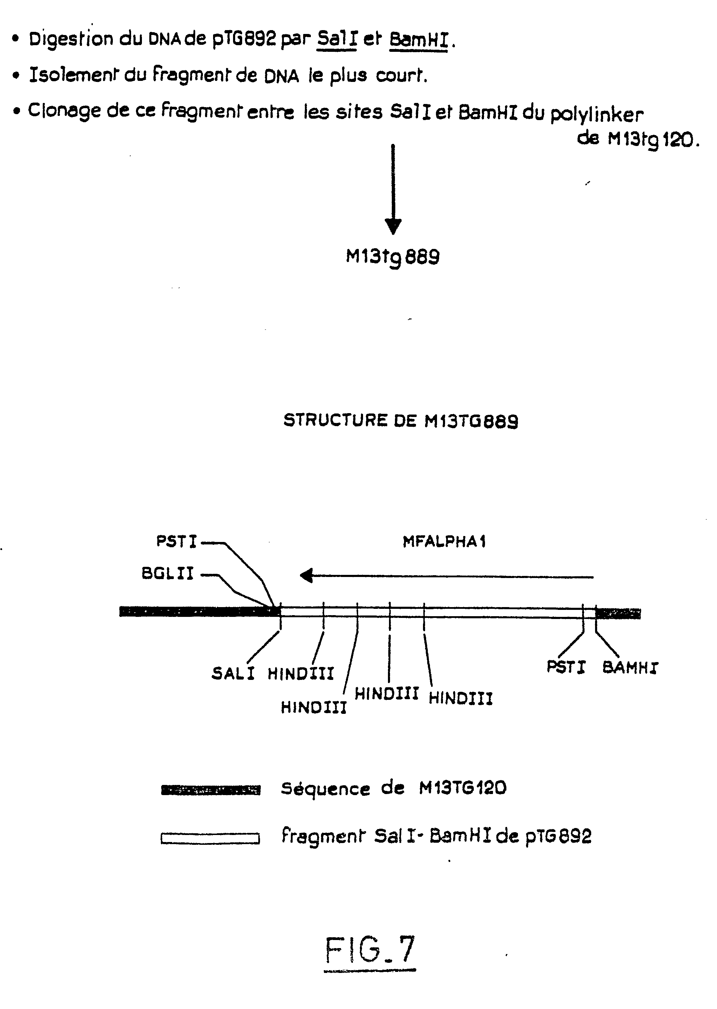

Le nouveau fragment BamHI-SalI ainsi obtenu a été cloné entre les sites BamHI et SalI du phage M13TG120 pour donner le phage M13TG889 (figure 7).

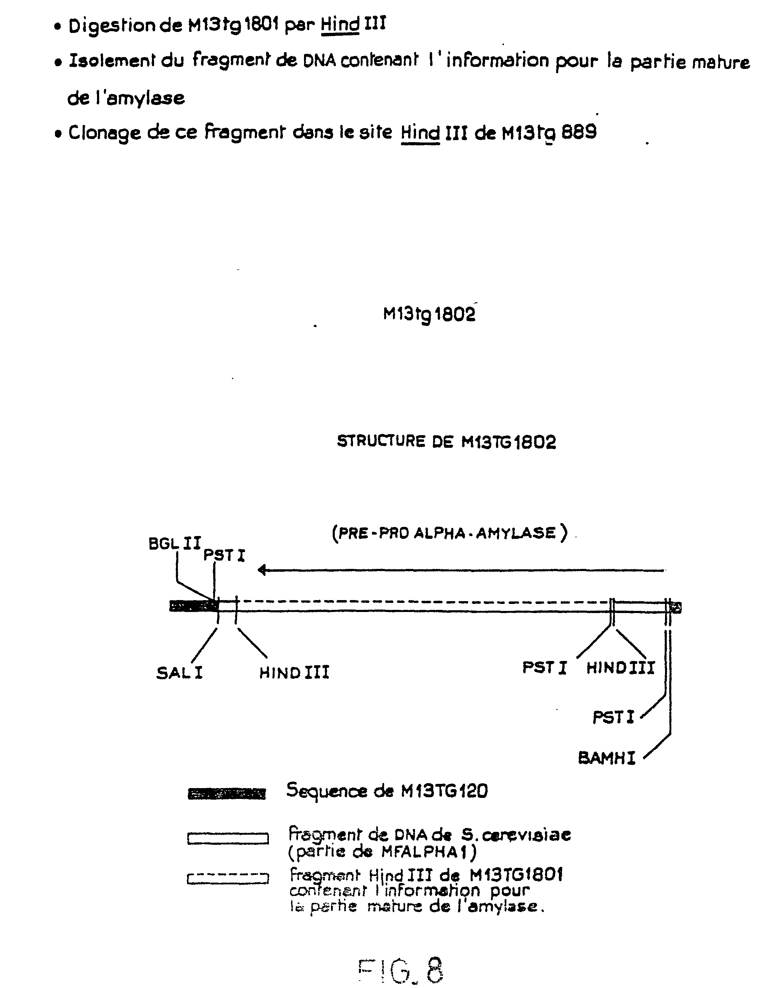

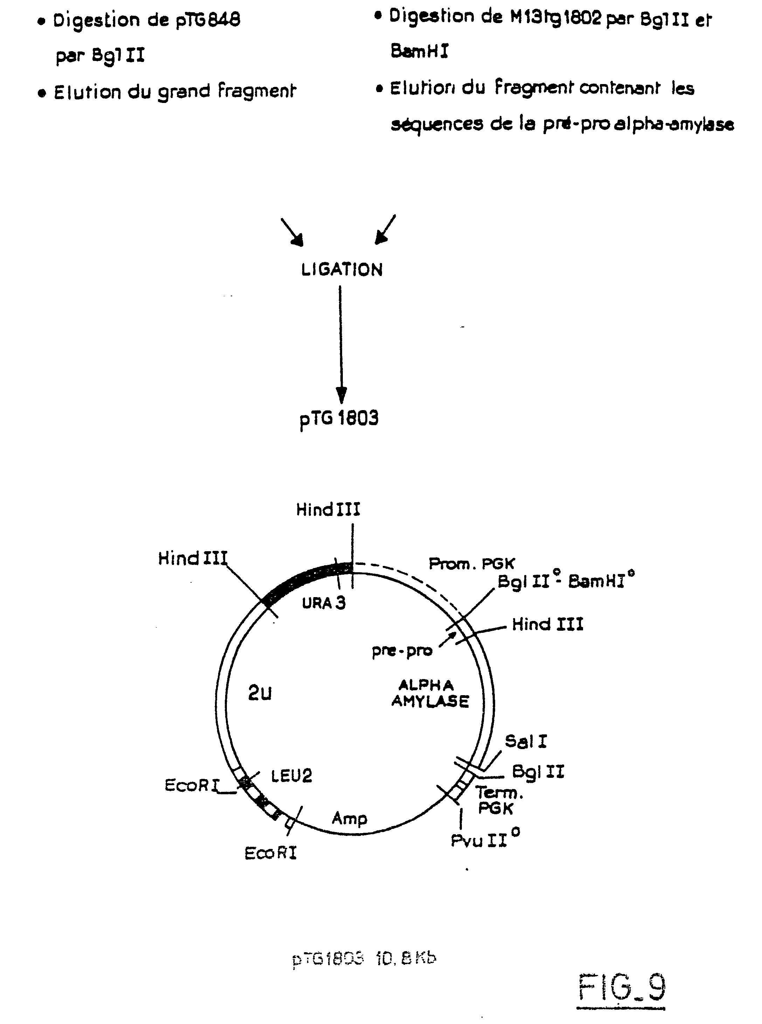

Le fragment HindIII de M13TG1801 (voir figure 2) comprenant, dans l'ordre, une courte séquence provenant de M13TG120 suivi de l'essentiel de la séquence de l'alpha-amylase (à partir du site PstI) a été cloné dans le site HindIII de M13TG889 de façon à mettre en phase la séquence de l'alpha-amylase avec la séquence pre-pro de la phéromone alpha. Le phage ainsi constitué est appelé M13TG1802 (figure 8). Le fragment BamHI-BglII de M13TG1802 a été échangé avec le fragment BglII-BglII de pTG848. On obtient ainsi un nouveau plasmide appelé pTG1803 (figure 9).

En résumé, le plasmide pTG1803 possède :

1) un fragment du plasmide 2μ de S. cerevisiae qui permet sa réplication dans Saccharomyces ;

2) le gène URA3[+] de S. cerevisiae qui permet la sélection des cellules transformées à partir d'une réceptrice ura3[-] sur milieu dépourvu de source de pyrimidine ;

3) l'origine de réplication du plasmide pBR322 et le gène de résistance à l'ampicilline qui permettent la réplication du plasmide et la sélection des transformés chez E. coli ;

4) le promoteur et le terminateur du gène PGK de levure qui assurent respectivement l'initiation et l'arrêt de transcription du bloc d'expression de l'exo-alpha-amylase ;

5) un gène codant pour un précurseur de cette exo-alpha-amylase constitué des séquences pre-pro du gène de la phéromone alpha-1 (MFα1) de S. cerevisiae fusionné à la séquence codant pour la partie mature de l'alpha-amylase.

EXEMPLE 3

TRANSFORMATION D'UNE SOUCHE DE LABORATOIRE AVEC L'ADN DE pTG1803

Ce plasmide, quand on transforme une souche Saccharomyces et sélectionne pour ura[+] ou leu[+], se réplique dans les cellules et confère à la souche la capacité de sécréter l'alpha-amylase. Comme exemple, on a transformé la souche TGY2sp13b (Matα ura3-251-373-328 leu2-3-12) et sélectionné pour ura[+] suivant les techniques décrites (19) et testé la présence d'alpha-amylase autour des colonies. La technique est la suivante :

On dépose un pâté de cellules sur boîtes YNBG standard supplémenté en amidon 2 % (poids/volume) (YNBG : yeast nitrogen base w/o aminoacides (Difco) 0,67 %, casamino-acides 0,5 %, glucose 2 % (poids/volume). Après 48 heures de croissance, on coule 1 ml de mélange aqueux iodé 3 g/l, KI 15 g/l pour colorer les boîtes. Le surplus de liquide est jeté.

Comme attendu, on a trouvé que tous les transformés sécrètent de l'alpha-amylase active.

## EXEMPLE 4

## CONSTRUCTION D'UN PLASMIDE D'INTEGRATION POUR S. CEREVISIAE

Pour ce faire, on a cloné dans le site BamHI d'un phage M13 (M13TG701) un fragment BamHI correspondant à la région du chromosome XV portant le gène HIS3 de S. cerevisiae (20). Ce nouveau phage est appelé M13TG1808.

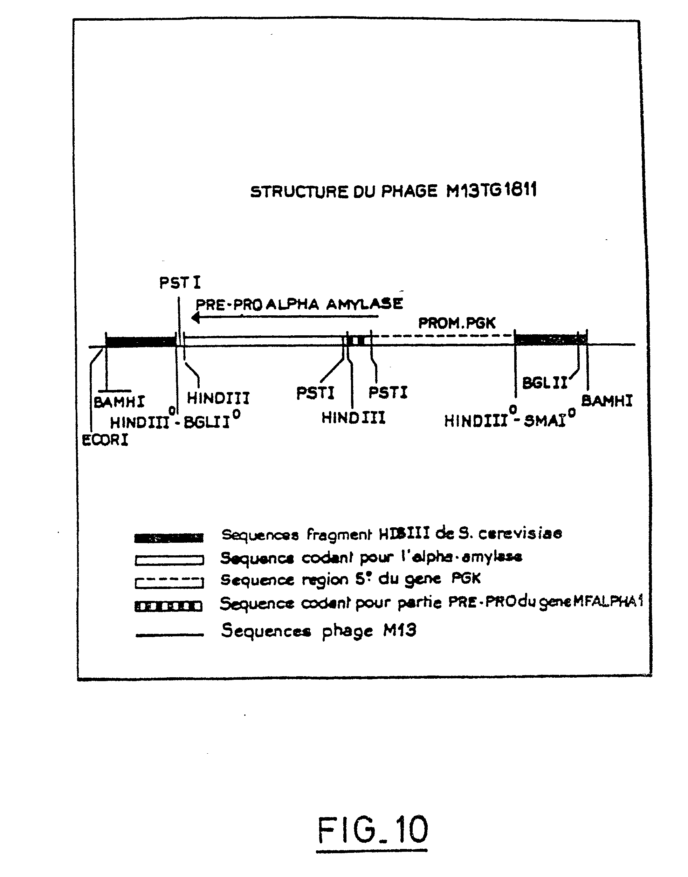

l'ADN de M13TG1808 a été digéré par HindIII ce qui libère un petit fragment d'environ 0,2 kb interne au gène His3. A la place de ce petit fragment, on a inséré par "blunt-end ligation", après action de la polymérase Klenow, le fragment SmaI-BglII de pTG1803 qui comprend la séquence du gène pre-pro-amylase sous contrôle du promoteur PGK. Ce nouveau phage, appelé M13TG1811, libère par BamHI un fragment d'ADN de levure dans lequel on a intégré le système d'expression de l'alpha-amylase (figure 10).

## EXEMPLE 5

## COTRANSFORMATION DE TGY2sp13b EN VUE D'OBTENIR DES LEVURES S. CEREVISIAE AYANT INTEGRE LE BLOC D'EXPRESSION DE L'ALPHA-AMYLASE

Le fait de libérer par digestion un fragment d'ADN linéaire homologue à une région du génome de levure favorise les évènements de recombinaison entre la région chromosomique et le fragment. En particulier, si le fragment renferme des séquences étrangères, on peut obtenir la substitution de la séquence chromosomique par celle du fragment linéaire et donc l'intégration des séquences étrangères dans le chromosome.

On a transformé la souche TGY2sp13b (Matα ura3-251-373-328 leu2-3-12) en ura[+] avec de l'ADN de pFL1 (21). Tout autre ADN plasmidique se répliquant dans la levure et comportant le gène ura3[+], à l'exclusion de ceux portant HIS3[+], pourrait être utilisé. En même temps que cet ADN de pFL1, on a ajouté aux cellules compétentes de l'ADN de M13TG1811 préalablement coupé par BamHI.

Plusieurs conditions ont été utilisées qui font varier les quantités d'ADN transformant. Les résultats sont indiqués dans le tableau I. Sur les 534 clones obtenus, on a testé ceux qui étaient devenus his[-].

En effet, la substitution du locus HIS3 par la séquence du fragment BamHI de pTG1811 entraîne une cassure de la séquence du gène HIS3 qui ne code plus pour une imidazole glycérate phosphate déshydratase fonctionnelle.

On a obtenu deux transformés his[-]. Ces deux clones (appelés TGY2sp13b amy1 et TGY2sp13b amy2 respectivement) produisent un halo de décoloration au test $I_2$-KI, ce qui indique qu'ils produisent de l'alpha-amylase. La comparaison de la taille du halo dans le cas des transformés intégrés et des transformés plasmidiques indique qu'on a une meilleure production d'alpha-amylase dans le cas de transformés plasmidiques.

Tableau I

| ADN de pFL1 | M13TG1811 digéré par BamHI | Nombre de clones obtenus |
|---|---|---|
| 0,1 µg | 1 µg | 180 |
| 0,5 µg | 5 µg | 152 |
| 1 µg | 10 µg | 202 |
| | TOTAL | 534 |

## EXEMPLE 6

## ECHANGE INVERSE

La souche TGY2sp13b amy1 a été directement transformée en his$^+$ par l'ADN de M13TG1808 découpé par BamHI. Sur 57 clones His$^+$, 38 ne faisaient plus de halo, signe qu'on avait réalisé l'expérience inverse de celle décrite dans l'exemple précédent.

## EXEMPLE 7

## INTEGRATION DANS UNE SOUCHE POLYPLOIDE DE BOULANGERIE (TGF1)

On a réalisé la même expérience que celle décrite dans l'exemple 5 en utilisant une souche utilisée dans l'industrie pour la fabrication du pain, fabriquée par la société FALA et commercialisée sous la marque "FALA". Cette souche étant polyploïde et prototrophe, la cotransformation avec M13TG1811 se fait en utilisant un plasmide conférant un phénotype dominant. On a utilisé la résistance au G418. Le plasmide que l'on a utilisé est un dérivé du plasmide pTG861 décrit dans le brevet français 84 04453. Le plasmide pTG825 ne diffère de pTG861 que par l'absence d'un ATG hors phase qui a été délété par mutagénèse in vitro pour permettre une meilleure expression du gène de résistance au G418. Tout autre plasmide conférant un phénotype dominant dans la levure pourrait être utilisé.

On a transformé la souche TGF1 avec de l'ADN de pTG1825 et de l'ADN de M13TG1811 préalablement digéré par BamHI comme décrit dans l'exemple 5, mais en remplaçant l'ADN de pFL1 par celui de pTG1825. On a obtenu 143 clones, dont 1 exprime l'alpha-amylase de façon stable au cours des générations (TGF amy1).

## EXEMPLE 8

## CONSTRUCTION D'UN VECTEUR D'INTEGRATION POUR UNE SOUCHE DE BRASSERIE (TGB1, S. UVARUM)

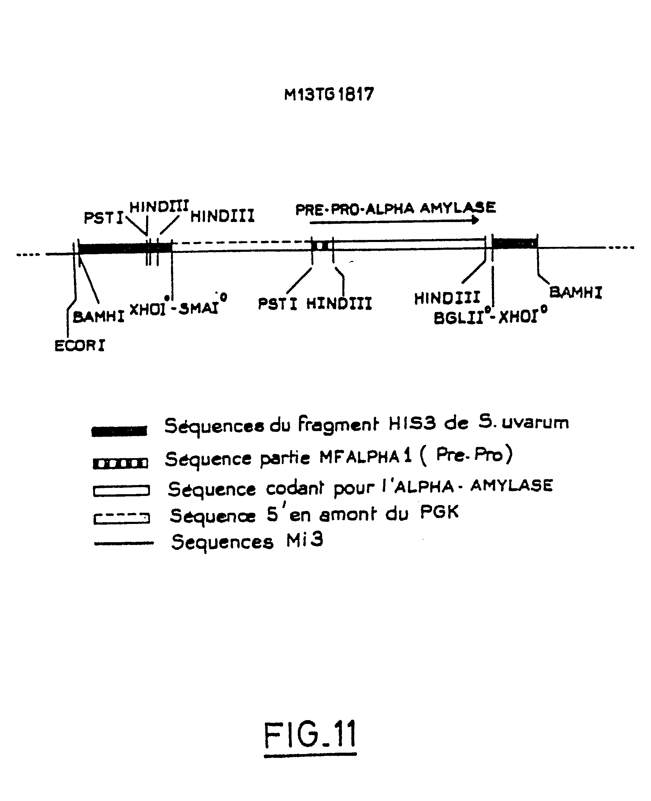

Un fragment BamHI de l'ADN chromosomique d'une souche de brasserie (TGB1) a été isolé sur la base de la complémentation de l'auxotrophie his⁻ de la souche de levure TGYsp1 (Mata ura3-251-373-328 his3-16-15) suivant les techniques classiques d'isolement de gènes de levure. Ce fragment BamHI a été cloné au site BamHI du phage M13TG701. Il présente un site XhoI unique que l'on a rendu franc par action de la polymérase klenow. Dans ce site XhoI modifié, on a intégré le bloc d'expression de l'exo-alpha-amylase sous forme d'un fragment SmaI-BglII provenant de pTG1803 et dont les extrémités ont été rendues franches (exemples 2 et 4). Le phage ainsi obtenu a été nommé M13TG1817 (figure 11).

EXEMPLE 9

INTEGRATION DU BLOC D'EXPRESSION DE L'EXO-AMYLASE DANS LE CHROMOSOME DE LA SOUCHE DE BRASSERIE

La technique de cotransformation a été appliquée à la souche TGB1. On a utilisé comme ADN plasmidique le pTG861 et l'ADN de M13TG1817 linéarisé par BamHI. La transformabilité de la souche TGB1 étant relativement mauvaise, la technique de Ito et al. est inefficace pour cette souche. On a donc utilisé une autre technique dérivée de Dickson et al. (22).

La technique que l'on a utilisée diffère de celle de Dickson et al. dans la manière d'ajouter l'agent sélectif : environ 15 heures après la transformation, on ajoute une surcouche de milieu complet (10 ml) contenant de la généticine (DIFCO) de façon à ce que la concentration finale de l'antibiotique soit 300 µg/ml dans la boîte. Cette technique a permis d'isoler 362 colonies résistantes au G418 dont 5 exprimaient l'amylase de manière stable.

## REFERENCES BIBLIOGRAPHIQUES

1. Schekman R. et P. Novick in the Molecular Biology of the yeast saccharomyces. Metabolism and Gene expression CHS 1982 New York pp 361-393.

2. Yamashita I., Itoh T., Fukui S., (1985) Agric. Biol. Chem. 49 pp 3089-3091.

3. Rothstein S.J., Lazarus C.M., Smith W.E., Baulcombe D.C., Catenby A.H. (1984) Nature 308 pp 662-665.

4. Thomser K.K. (1983) Carslberg Res. Commun 78 pp 545-555.

5. Innis M.A., Holland, M.J., Mc Cake P.C., et al Science 228 pp 21-26.

6. Yamashita, I., Itoh, T., Fukui, S., (1985) Appl. Microbiol. Biotechnol. 23 : 130-133.

7. Rothstein R.J., (1983) Methods Enzymol. 101 p 202.

8. Bussière G., De La Guérivière Ann. Technol. Agric. (1974) 23 pp 175-189.

9. Scherer, G., Telford, J., Baldari, C. and Pirrotta, V. : Isolation of cloned genes differentially expressed at early and late stages of Drosophila embryonic development. Develop. Biol. 86 (1981) 438-447.

10. Gryczan, T., Shivakumar, A.G. and Dubnau, D. : Characterization of chimeric plasmid cloning vehicles in Bacillus Subtilis. J. Bacteriol. 141 (1980) 246-253.

11. Beggs, J. (1981) Genetic Engineering 2, 175-203.

12. Broach, J.R. (1981) in the Molecular Biology of the yeast Saccharomyces. Life cycle and Inheritance CHS press, New York.

13. Bach, M.L., Lacroute, F. et Botstein D. (1979) Proc. Natl. Acad. Sci. (USA) 76, 386-390.

14. Sutcliffe, J.G. : Proc. Natl. Acad. Sci. USA 75 (1978) 3737-3741.

15. Hitzeman R.A., Hagie, E.F., Hayfflick, J.S. et al. (1982) Nucleic Acids Res. 10, 7791-7808.

16. Maniatis, T., Fritsch, E.F., et Sambrook, J. (1982) C.S.H. Laboratory New York.

17. Cohen, N., Chiang, A.C.-Y. Hsu, L. 1972 Proc. Natl. Acad. Sci USA 69 : 2110-2114.

18. Lathe, R.F., J.P. Lecocq, R. Everett Genetic Engineering 4, Academie Press.

19. Ito, H., Fukuda Y., Murata, K., et Kimura, A., (1983) J. Bacteriol. 153, 163-168.

20. Struhl, K., (1985) Nucl. Acids Research 13 8587-8601.

21. Chevallier, M.R., Bloch, J.C., Lacroute, F. Gene (1980) 11,11-19.

22. Webster, T.D., & R.C. Dickson Gene (1983) 26 243-252.

## REVENDICATIONS

1) Souche de Saccharomyces caractérisée en ce qu'elle comporte un bloc d'expression de l'exo-alpha-amylase intégré dans un chromosome ou bien inséré sur un vecteur extrachromosomique.

2) Souche selon la revendication 1, caractérisée en ce que l'exo-alpha-amylase est une exo-alpha-amylase thermostable de Bacillus licheniformis.

3) Souche selon l'une des revendications 1 et 2, caractérisée en ce que le bloc d'expression de l'exo-alpha-amylase à la structure suivante :

$$\text{Pr} \qquad \text{Pre Pro} \qquad \text{alpha-amylase}$$

Pr est un promoteur de levure,

alpha-amylase est la séquence codant pour la partie mature de l'alpha-amylase,

pre pro est la séquence pre-pro du gène de la phéromone alpha-1.

4) Souche selon l'une des revendications 1 à 3, caractérisée en ce que Pr est le promoteur du gène PGK.

5) Souche selon l'une des revendications 1 à 4, caractérisée en ce que l'extrémité 3' comporte une séquence de terminateur de levure.

6) Souche selon la revendication 5, caractérisée en ce que la séquence de terminateur de levure est celle du gène PGK.

7) Souche selon l'une des revendications 1 à 6, caractérisée en ce que le vecteur extrachromosomique est un plasmide portant une origine de réplication dans les levures.

8) Souche selon la revendication 7, caractérisée en ce que l'origine de réplication est l'origine de réplication du plasmide 2µ.

9) Souche de Saccharomyces selon l'une des revendications 1 à 8, caractérisée en ce que la souche est une souche de Saccharomyces sauvage.

10) Souche selon l'une des revendications 1 à 8, caractérisée en ce qu'il s'agit d'une souche polyploïde de boulangerie.

11) Souche selon la revendication 10, caractérisée en ce que la souche polyploïde de boulangerie est la souche TGF1.

12) Souche selon l'une des revendications 1 à 8, caractérisée en ce que la souche est une souche de brasserie.

13) Souche selon la revendication 12, caractérisée en ce que la souche est une souche de S. uvarum.

14) Procédé de préparation d'une alpha-amylase thermostable, caractérisé en ce qu'on cultive sur un milieu de culture une souche de Saccharomyces selon l'une des revendications 1 à 13 et on récupère l'enzyme dans le milieu de culture.

15) Application des levures selon l'une des revendications 1 à 13 pour l'intégration d'une séquence d'ADN déterminée dans ladite souche, caractérisée en ce que le vecteur d'intégration comporte la séquence d'ADN à intégrer entre des séquences assurant l'échange avec le bloc d'expression de l'exo-alpha-amylase ou une partie essentielle de ce bloc, et en ce que l'on sélectionne les souches ne produisant plus de halo de décoloration en présence d'$I_2$ et d'amidon.

FIG_1

pTG443

- Digestion par _Pst1_

- Elution du fragment _Pst1_ le plus court

- Clonage au site _Pst1_ de M13tg112

M13tg1801

M13tg1801

FIG_2

■ Sequence de M13tg112

☐ Segment de DNA (PstI) de pTG443

SMA I   MIND III

MIND III

URA3   PGK 5'
+PROM

BGL II

PGK

SAL I

2U

PTG 848
10.35KB

PST I⁰
ECOR I   LEU2

PCK
TERM

ECOR I

PST I⁰
ECOR I

AMP^R   ORI   BGL II

PVU II⁰

MIND III⁰

PST I   PVU II⁰

digestion AVEC Hind III
TRAITEMENT KLENOW

ligation en présence de Hind III

transformation dans E.coli , pyr F⁻

SMA I   MIND III⁰

MIND III⁰

URA3   PGK 5'
+PROM

BGL II

PGK

SAL I

2U

PTG 874
10.35KB

PST I⁰
ECOR I   LEU2

PGK
TERM

ECOR I

PST I⁰
ECOR I

AMP^R   ORI   BGL II

PVU II⁰

MIND III⁰

PST I   PVU II⁰

FIG. 3

SMA1°/ECOR1°
BGLII
PST I
PST I
HINDIII (X4)
SAL I
HINDIII
URA3
5'
PROM
MF
ALPHA1
2U
PTG 876
9,65 KB
PVUII°/ECORI
PGK
TERM
BGLII
ORI
PVUII°/HIND III°
PST I°
LEU 2
ECOR I
APR
PST I°
PST I
ECORI

• DIGESTION PARTIELLE BGLII
• KLENOW
• LIGATION

SMA1°/ECOR1°
BGLII
PST I
PST I
HINDIII (X4)
SAL I
HINDIII
URA3
5'
PROM
MFALPHA1
2u
PTG 883
9,65 KB
PVUII°/ECORI
PGK
TERM
BGLII°
ORI
PVUII°/HIND III°
PST I°
LEU 2
ECOR I
APR
PST I°
ECORI

FIG_5

FIG.6

CONSTRUCTION DE PTG 892 A PARTIR DE PTG 883

- Digestion du DNA de pTG892 par <u>SalI</u> et <u>BamHI</u>.
- Isolement du fragment de DNA le plus court.
- Clonage de ce fragment entre les sites SalI et BamHI du polylinker de M13tg 120.

M13tg 889

STRUCTURE DE M13TG889

Séquence de M13TG120

fragment SalI- BamHI de pTG892

FIG_7

- Digestion de M13tg1801 par <u>Hind</u> III
- Isolement du fragment de DNA contenant l'information pour la partie mature de l'amylase
- Clonage de ce fragment dans le site <u>Hind</u> III de M13tg 889

M13tg1802

STRUCTURE DE M13TG1802

(PRE-PRO ALPHA-AMYLASE)

BGL II  PST I

SAL I        HIND III              PST I   HIND III

PST I

BAMHI

▬▬▬ Sequence de M13TG120

☐─────☐ Fragment de DNA de S. cerevisiae
(partie de MFALPHA1)

⌐─────⌐ Fragment Hind III de M13TG1801
contenant l'information pour
la partie mature de l'amylase.

FIG. 8

- Digestion de pTG848 par Bgl II
- Elution du grand fragment

- Digestion de M13tg1802 par Bgl II et BamHI
- Elution du fragment contenant les séquences de la pré-pro alpha-amylase

LIGATION

pTG 1803

pTG1803 10.8Kb

FIG_9

STRUCTURE DU PHAGE M13TG1811

FIG_10

M13TG1817

PRE·PRO·ALPHA AMYLASE

PSTI HINDIII HINDIII

BAMHI XHOI°-SMAI°   PSTI HINDIII   HINDIII   BAMHI
                                    BGLII°-XHOI°
ECORI

▬▬▬  Séquences du fragment HIS3 de S. uvarum

▭▭▭  Séquence partie MFALPHA1 ( Pre·Pro)

▭▭  Séquence codant pour l'ALPHA·AMYLASE

▭--▭  Séquence 5'en amont du PGK

▬▬▬  Sequences Mi3

# FIG.11

**Office européen des brevets**

## RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| X | BIOTECHNOLOGY, vol. 4, no. 4, avril 1986, pages 311-315, New York, US; S.A. FILHO et al.: "Stable yeast transformants that secrete functional alpha-amylase encoded by cloned mouse pancreatic cDNA" * En entier * | 1,3,5, 7-9 | C 12 N 15/00 C 12 N 1/16 C 12 N 1/18 C 12 N 9/28 |
| Y | Idem | 2,4,6, 10-15 | |
| | --- | | |
| X | CHEMICAL ABSTRACTS, vol. 104, no. 5, février 1986, pages 160-161, résumé no. 29836d, Columbus, Ohio, US; & JP-A-60 168 387 (KAGAKU OYOBI KESSEI RYOHO KENKYUSHO) 31-08-1985 * Résumé * | 1,7-9 | |

|  |  |  | DOMAINES TECHNIQUES RECHERCHES (Int Cl 4) |
|---|---|---|---|
| | --- | | |
| X | CHEMICAL ABSTRACTS, vol. 104, no. 15, avril 1986, page 183, résumé no. 124190x, Columbus, Ohio, US; T.F. GOROZHANKINA et al.: "Expression of the alpha-amylase gene of Bacillus amyloliquefaciens in the yeast Saccharamyces cerevisiae", & DOKL. AKAD. NAUK SSSR 1985, 285(3), 717-20 [Biochem.] * Résumé * | 1,7-9 | C 12 N C 12 P |

--- -/-

Le présent rapport de recherche a été établi pour toutes les revendications

| Lieu de la recherche | Date d'achèvement de la recherche | Examinateur |
|---|---|---|
| LA HAYE | 18-08-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

**Office européen des brevets**

## RAPPORT DE RECHERCHE EUROPEENNE

### DOCUMENTS CONSIDERES COMME PERTINENTS

| Catégorie | Citation du document avec indication, en cas de besoin. des parties pertinentes | Revendication concernée | CLASSEMENT DE LA DEMANDE (Int Cl 4) |
|---|---|---|---|
| X | AGRICULTURAL AND BIOLOGICAL CHEMISTRY, vol. 49, no. 10, octobre 1985, pages 3089-3091, Tokyo, JP; I. YAMASHITA et al.: "Cloning and expression of the Saccharomycopsis fibuligera alpha-amylase gene in Saccharomyces cerevisiae" * En entier * | 1,9 | |
| X | NATURE, vol. 308, no. 5960, 12 avril 1984, pages 662-665, Reading, Berks, GB; S.J. ROTHSTEIN et al.: "Secretion of a wheat alpha-amylase expressed in yeast" * En entier * | 1,7-9 | |
| P,X | WO-A-8 603 778 (BREWING RESEARCH FOUNDATION) * Revendications 8 * | 1,4,12 | DOMAINES TECHNIQUES RECHERCHES (Int Cl.4) |
| X | CA-A-1 192 150 (UNIVERSITY OF CALIFORNIA) * Page 8, ligne 6 - page 9, ligne 2; page 17, lignes 5-9; revendication 9 * | 1,7,8, 14 | |
| A | FR-A-2 537 602 (CNRS) * En entier * | 2,14 | |

-/-

Le présent rapport de recherche a été établi pour toutes les revendications

| Lieu de la recherche | Date d'achèvement de la recherche | Examinateur |
|---|---|---|
| LA HAYE | 18-08-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OE B Form 1503 03 82

Office européen
des brevets

**RAPPORT DE RECHERCHE EUROPEENNE**

Numero de la demande

EP 87 40 1050

Page 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| Y | EP-A-0 034 470 (CPC INTERNATIONAL) <br> * Page 3, ligne 18 - page 4, ligne 3; page 7, lignes 17-23; page 11, lignes 5-13; page 32, lignes 10-28 * <br><br> --- | 2,14 | |
| Y | EP-A-0 166 659 (INRA) <br> * Page 5, lignes 27-34; page 12, lignes 13-19 * <br><br> --- | 2,14 | |
| Y | EP-A-0 163 491 (BIOTECHNICA) <br> * Page 3, lignes 6-11; page 4, ligne 30 - page 5, ligne 12 * <br><br> --- | 10-13 | |
| Y | GENE, vol. 36, no. 1/2, 1985, pages 87-95, Esevier Science Publishers, Amsterdam, NL; H. RUDOLPH et al.: "One-step gene replacement in yeast by cotransformation" <br> * Page 88, colonne de gauche, paragraphe 2; page 89, colonne de gauche, partie a); figure 1; page 93, colonne de droite, paragraphe 3 * <br><br> ---     -/- | 15 | DOMAINES TECHNIQUES RECHERCHES (Int Cl 4) |

Le présent rapport de recherche a été établi pour toutes les revendications

| Lieu de la recherche | Date d'achèvement de la recherche | Examinateur |
|---|---|---|
| LA HAYE | 18-08-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

0252774

Office européen
des brevets

RAPPORT DE RECHERCHE EUROPEENNE

Numéro de la demande

EP 87 40 1050

Page 4

## DOCUMENTS CONSIDERES COMME PERTINENTS

| Catégorie | Citation du document avec indication. en cas de besoin. des parties pertinentes | Revendication concernée | CLASSEMENT DE LA DEMANDE (Int. Cl.4) |
|---|---|---|---|
| Y | BIOTECHNOLOGY AND GENETIC ENGINEERING REVIEWS, vol. 3, septembre 1985, pages 377-416, Intercept Ltd; S.M. KINGSMAN et al.: "Heterologous gene expression in Saccharomyces cerevisiae" * Page 382, dernier paragraphe - page 384, premier paragraphe; page 389, dernier paragraphe - page 390, figures 5,6; page 403, lignes 1-8; page 406, trois dernières lignes * | 3-8 | |
| P,Y | GENE, vol. 43, no. 1/2, 1986, pages 85-94, Elsevier Science Publishers B.V., Amsterdam, NL; H. SHIMOTSU et al.: "Construction of a single-copy integration vector and its use in analysis of regulation of the trp operon of Bacillus subtilis" * Whole document * | 15 | |
| A | FR-A-2 533 583 (CNRS) * Revendications 1,3 * | 2 | |
| A | WO-A-8 404 539 (TRANSGENE) * Revendications 8-12,23,25,26 * | 3-8 | |

--- -/-

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 18-08-1987 | MADDOX A.D. |

# RAPPORT DE RECHERCHE EUROPEENNE

Office européen
des brevets

| | DOCUMENTS CONSIDERES COMME PERTINENTS | | Page 5 | |
|---|---|---|---|---|
| Catégorie | Citation du document avec indication, en cas de besoin, des parties pertinentes | Revendication concernée | CLASSEMENT DE LA DEMANDE (Int Cl 4) | |
| A | CHEMICAL ABSTRACTS, vol. 97, no. 1, juillet 1982, page 167, résumé no. 1562z, Columbus, Ohio, US; A. NASIM et al.: "Use of recombinant DNA techniques for the development of amylolytic strains of Saccharomyces cerevisiae for alcohol production", & PROC. - BIOENERGY R & D SEMIN. 1981, 3rd, 141-3 | | | |
| A | CHEMICAL ABSTRACTS, vol. 98, no. 7, février 1983, page 179, résumé no. 47922r, Columbus, Ohio, US; R.A. IRVING et al.: "Development of an amylolytic Saccharomyces cerevisiae by genetic engineering", & PROC. - BIOENERGY R & D SEMIA 1982, 4th, 541-5 | | DOMAINES TECHNIQUES RECHERCHES (Int Cl 4) | |
| A | FOOD TECHNOLOGY, vol. 38, no. 2, février 1984, pages 99-106+111, Chicago, Illinois, US; C.J. PANCHAL et al.: "Genetic manipulation of brewing and related yeast strains" --- -/- | | | |
| | Le présent rapport de recherche a été établi pour toutes les revendications | | | |
| | Lieu de la recherche LA HAYE | Date d achèvement de la recherche 18-08-1987 | Examinateur MADDOX A.D. | |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

0252774

Numéro de la demande

Office européen
des brevets

**RAPPORT DE RECHERCHE EUROPEENNE**

EP 87 40 1050

Page 6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| A | CHEMICAL ABSTRACTS, vol. 82, no. 25, 23 juin 1975, page 390, résumé no. 169018r, Columbus, Ohio, US; G. BUSSIERE et al.: "Use of alpha-amylases and glucamylase in bakery" & ANN. TECHNOL. AGRIC. 1974, 23(2), 175-89 | | |
| A | --- APPLIED AND ENVIRONMENTAL MICROBIOLOGY, vol. 47, no. 5, mai 1984, pages 1164-1166, American Society for Microbiology; C.J. PANCHAL et al.: "Susceptibility of Saccharomyces spp. and Schwanniomyces spp. to the aminoglycoside antibiotic G418" | | |
| A | --- CHEMICAL ABSTRACTS, vol. 96, 1982, page 482, résumé no. 50642p, Columbus, Ohio, US; J.R. HUDSON: "Recent research at the BRF", & PROC. CONV. - INST. BREW. (AUST. N. Z. SECT.) 1980, 16th, 7-18 * Résumé * | | **DOMAINES TECHNIQUES RECHERCHES (Int. Cl.4)** |

Le présent rapport de recherche a été établi pour toutes les revendications

| Lieu de la recherche | Date d'achèvement de la recherche | Examinateur |
|---|---|---|
| LA HAYE | 18-08-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82